(19)

Europäisches
Patentamt
European
Patent Office
Office européen
des brevets

(11) **EP 3 868 315 A1**

(12) **EUROPEAN PATENT APPLICATION**

(43) Date of publication:
   **25.08.2021 Bulletin 2021/34**

(51) Int Cl.:
   ***A61B 17/32*** *(2006.01)*

(21) Application number: **21154898.7**

(22) Date of filing: **04.02.2021**

(84) Designated Contracting States:
   **AL AT BE BG CH CY CZ DE DK EE ES FI FR GB GR HR HU IE IS IT LI LT LU LV MC MK MT NL NO PL PT RO RS SE SI SK SM TR**
   Designated Extension States:
   **BA ME**
   Designated Validation States:
   **KH MA MD TN**

(30) Priority:  **18.02.2020  CN 202010099097**

(71) Applicant: **SMTP Medical Co., Ltd.**
   **Beijing 102600 (CN)**

(72) Inventors:
   • **Cao, Qun**
    **BEIJING, 102600 (CN)**
   • **Zhan, Songtao**
    **BEIJING, 102600 (CN)**
   • **Hu, Xiaoming**
    **BEIJING, 102600 (CN)**
   • **Dai, Zhiling**
    **BEIJING, 102600 (CN)**

(74) Representative: **Beyer, Andreas**
   **Wuesthoff & Wuesthoff**
   **Patentanwälte PartG mbB**
   **Schweigerstrasse 2**
   **81541 München (DE)**

(54) **ULTRASONIC SURGICAL SYSTEM**

(57)    Disclosed is an ultrasonic surgical system, comprising: an ultrasonic transducer used for converting an alternating current signal into vibration; and an ultrasonic amplitude transformer used for amplifying the amplitude of the vibration generated by the ultrasonic transducer, wherein the product $L_1 \times C_1$ of a dynamic equivalent inductance $L_1$ and a dynamic equivalent capacitance $C_1$ of the ultrasonic surgical system in a no-load state and the product $L' \times C'$ of a dynamic equivalent inductance $L'$ and a dynamic equivalent capacitance $C'$ of the ultrasonic surgical system in an on-load state

$$\left| 1 - \frac{\sqrt{L_1 \times C_1}}{\sqrt{L' \times C'}} \right| = \alpha$$

satisfy the relational expression                    ' such that a resonance frequency $f_s$ of the ultrasonic surgical system in the no-load state and a resonance frequency $f_s'$ thereof in the on-load state satisfy the relational expression

$$\left| 1 - \frac{f_s'}{f_s} \right| = \alpha$$

' wherein $\alpha$ is less than or equal to 0.1%, and preferably $\alpha$ is less than or equal to 0.05%.

*Fig. 1*

EP 3 868 315 A1

**Description**

Technical Field

The present disclosure relates to an ultrasonic surgical system, and specifically to an ultrasonic surgical system for a surgeon to perform incision and/or coagulation.

Background

An ultrasonic surgical system, such as an ultrasonic hemostatic scalpel, has been extensively applied to surgery, with an end effector of such instrument being started or excited by an ultrasonic frequency, for example, the hemostatic scalpel induces longitudinal vibration or longitudinal and torsional coupled vibration, which produces local thermal energy and vibrational energy in the clamped tissue, thereby facilitating coagulation hemostasis and incision.

The ultrasonic surgical system generally comprises an ultrasonic transducer and an ultrasonic amplitude transformer, wherein the ultrasonic transducer is used for converting an alternating current signal into vibration energy of piezoelectric ceramic, and the ultrasonic amplitude transformer further amplifies the amplitude of the piezoelectric ceramic and then drives, under a condition of certain pressure or clamping pressure, a tip part of the amplitude transformer to perform incision and coagulation operation.

For an ultrasonic surgical system in the prior art, the system design thereof does not take into account the influence of a load (such as the type of a target tissue to be incised/coagulated and/or the magnitude of an applied clamping pressure) on the resonance frequency of the ultrasonic surgical system. When the ultrasonic surgical system performs operation (such as incision and/or coagulation) on a target tissue, due to the interaction between the load and the ultrasonic surgical system, the actual resonance frequency (including: the resonance frequency in an on-load state (the resonance frequency when only clamping pressure is applied to the tissue, or the resonance frequency when clamping pressure is applied to the tissue and power smaller than or equal to 1 W is output), i.e., the resonance frequency when clamping pressure is applied to the target tissue; and the resonance frequency during the operation in an on-load state (the resonance frequency when clamping pressure is applied to the tissue and power for incision and/or coagulation of the tissue is output), i.e., the resonance frequency that the ultrasonic surgical system has, after the temperature at the target tissue and the incision part significantly increases due to the interaction between the load and the ultrasonic surgical system, when the ultrasonic surgical system performs incision and/or coagulation operation on the target tissue) of the ultrasonic surgical system deviates from the designed resonance frequency (the resonance frequency in a no-load state (i.e., no clamping pressure is applied to the tissue)), causing the node position (the position of an integer multiple of a half-wavelength, where a boss is provided and engaged to an external support sleeve) obtained according to the resonance frequency in a no-load state to be inconsistent with the position in actual work, such that when the ultrasonic surgical system is driven at the designed resonance frequency, the boss at the node position and the external support sleeve coming into contact with the boss will generate violent vibration therebetween and generate heat by friction, which not only results in the increased loss of the ultrasonic surgical system, but also may also have a risk factor to cause high-temperature burns in some tissues of the patient. In order to mitigate this risk, in the prior art, an elastic member will be machined on the boss by means of bonding, injection molding, or other processes to form a soft support between the ultrasonic amplitude transformer and the external support sleeve, but this will significantly increase the manufacturing cost of the ultrasonic surgical system.

In addition, the manufacturing accuracy, the assembly accuracy, etc. of the components of the ultrasonic surgical system may also cause the resonance frequency of the ultrasonic surgical system to deviate from the designed resonance frequency (the resonance frequency in a no-load state).

In the prior art, in order to solve the problem of deviation of the resonance frequency from the initially designed resonance frequency in actual work, closed-loop control is generally used to enable the ultrasonic transducer to drive the ultrasonic surgical system at the resonance frequency of the ultrasonic surgical system in actual work, such that the vibration frequency of the ultrasonic surgical system tracks or approaches as close as possible to the resonance frequency of the ultrasonic surgical system in actual work. For example, the Chinese invention patent applications CN 104582606 A and CN 104582629 A disclose that a phase-lock loop in a control system of a generator is used to monitor feedback from an acoustic assembly, and the phase-lock loop adjusts the frequency of electrical energy generated by a generator to match the resonance frequency of the longitudinal vibration mode selected by the acoustic assembly. For example, the Chinese invention patent application CN 110507389 A discloses that a DSP is used to achieve resonance frequency control, and the Chinese invention patent application CN 110448355 A discloses that a microcontroller, a digital signal processor, etc. are used to achieve resonance frequency control. For another example, the Chinese invention patent application CN 102458286 A discloses an ultrasonic surgical device, comprising: an ultrasonic vibrator that can generate ultrasonic vibration; a drive part that drives the ultrasonic vibrator according to a drive signal; a probe having a base end portion and a front end portion, the base end portion being kinematically coupled to the ultrasonic vibrator, and the probe being used for transferring the ultrasonic vibration generated by the ultrasonic vibrator from the base end portion to the front end portion so as to enable the front end portion to generate ultrasonic vibration for the treatment of biological tissues; and a resonance frequency tracking portion that automatically adjusts the frequency of the above drive signal such that the frequency of the drive signal tracks the resonance frequency of the ultrasonic vibrator so as to enable the front end portion to perform ultrasonic vibration at the resonance frequency.

It should be noted that although the closed-loop control is used in the prior art to make the vibration frequency of the ultrasonic surgical system track or approach as close as possible to the resonance frequency of the ultrasonic surgical system in actual work so as to improve the incision efficiency, etc., the ultrasonic surgical system of the existing design still has the technical problem caused by the mismatch between the resonance frequency in actual work and the designed resonance frequency, for example, the violent vibration between the boss provided at the node position and the external support sleeve.

In addition, although the closed-loop control is used in the prior art to make the vibration frequency of the ultrasonic surgical system track or approach as close as possible to the actual resonance frequency of the ultrasonic surgical system, since a surgical procedure is a dynamic procedure, i.e., in the surgical procedure, the load (such as the type of target tissue to be incised and/or coagulated and/or the magnitude of clamping pressure and/or the length of the clamped target tissue) changes constantly to cause constant change of the actual resonance frequency of the ultrasonic surgical system, the vibration frequency of the ultrasonic surgical system always has a large deviation from the actual resonance frequency of the ultrasonic surgical system.

Summary of the Invention

In order to at least partially overcome or mitigate one or more of the defects described above in the prior art, the present disclosure provides an ultrasonic surgical system, comprising: an ultrasonic transducer used for converting an alternating current signal into vibration; and an ultrasonic amplitude transformer used for amplifying the amplitude of the vibration generated by the ultrasonic transducer so as to drive a working part located at a terminal end of the ultrasonic amplitude transformer to perform incision and/or coagulation operation for a target tissue, wherein the product $L_1 \times C_1$ of a dynamic equivalent inductance $L_1$ and a dynamic equivalent capacitance $C_1$ of the ultrasonic surgical system in a no-load state and the product $L' \times C'$ of a dynamic equivalent inductance $L'$ and a dynamic equivalent capacitance $C'$ of the ultrasonic surgical system in an on-load state satisfy the following relational expression (1),

$$\left| 1 - \frac{\sqrt{L_1 \times C_1}}{\sqrt{L' \times C'}} \right| = \alpha \qquad (1)$$

such that a resonance frequency $f_s$ of the ultrasonic surgical system in the no-load state and a resonance frequency $f_s'$ thereof in the on-load state satisfy the following relational expression (2):

$$\left| 1 - \frac{f_s'}{f_s} \right| = \alpha \qquad (2)$$

wherein, $f_s = \frac{1}{2\pi\sqrt{L_1 \times C_1}}$, $f_s' = \frac{1}{2\pi\sqrt{L' \times C'}}$, and $\alpha$ is less than or equal to 0.1%, and preferably $\alpha$ is less than or equal to 0.05%.

According to the present disclosure, the product $L_1 \times C_1$ of the dynamic equivalent inductance $L_1$ and the dynamic equivalent capacitance $C_1$ of the ultrasonic surgical system in the no-load state and the product $L' \times C'$ of the dynamic equivalent inductance $L'$ and the dynamic equivalent capacitance $C'$ of the ultrasonic surgical system in the on-load state satisfy the relational expression (1) within an expected tissue clamping pressure range of the working part of the ultrasonic surgical system, such that the resonance frequency $f_s$ of the ultrasonic surgical system in the no-load state and the resonance frequency $f_s'$ thereof in the on-load state satisfy the relational expression (2) within the expected tissue clamping pressure range.

According to the present disclosure, the expected tissue clamping pressure range is 1 N to 40 N, preferably, the expected tissue clamping pressure range is 5 N to 36 N, and more preferably, the expected tissue clamping pressure range is 10 N to 36 N.

According to the present disclosure, the target tissue includes one or more soft tissues including tendons, ligaments, fascia, skin, connective tissue, fat, synovial membranes, muscles, nerves, and blood vessels.

According to the present disclosure, when operated in an on-load state (i.e., clamping pressure is applied to the tissue and power for incision and/or coagulation is output), the resonance frequency offset of the ultrasonic surgical system and the temperature of the working part of the ultrasonic surgical system satisfy a one-to-one functional relationship, such that the temperature of the working part of the ultrasonic surgical system can be determined only according to the resonance frequency offset of the ultrasonic surgical system.

According to the present disclosure, the transducer comprises a transducer housing defining an opening; at least two piezoelectric elements arranged in the opening; and a fastener engaged to the transducer housing to close the opening and applying a compressive force to the at least two piezoelectric elements such that, by means of adjusting any one or a combination of any two or more of the following parameters a, b and c of the transducer, the product $L_1 \times C_1$ of the dynamic equivalent inductance $L_1$ and the dynamic equivalent capacitance $C_1$ of the ultrasonic surgical system in the no-load state and the product $L' \times C'$ of the dynamic equivalent inductance $L'$ and the dynamic equivalent capacitance $C'$ of the ultrasonic surgical system in the on-load state satisfy the relational expression (1):

> a: the number of the piezoelectric elements;
> b: the diameter of the piezoelectric element; and
> c: a compressive force applied to the piezoelectric element.

According to the present disclosure, by means of adjusting any one or a combination of any two or more of the material, the length, the change of cross section along a longitudinal axis, and the change of cross section of the working part along the longitudinal axis of the ultrasonic amplitude transformer, the product $L_1 \times C_1$ of the dynamic equivalent inductance $L_1$ and the dynamic equivalent capacitance $C_1$ of the ultrasonic surgical system in the no-load state and the product $L' \times C'$ of the dynamic equivalent inductance $L'$ and the dynamic equivalent capacitance $C'$ of the ultrasonic surgical system in the on-load state satisfy the relational expression (1).

According to the present disclosure, the ultrasonic surgical system further comprises a support sleeve, the ultrasonic amplitude transformer is arranged in the support sleeve, the ultrasonic amplitude transformer is provided with at least one boss at a node position, the ultrasonic amplitude transformer is supported by the support sleeve at the at least one boss, and by means of adjusting the number of the bosses and/or the position of the bosses of the ultrasonic amplitude transformer, the product $L_1 \times C_1$ of the dynamic equivalent inductance $L_1$ and the dynamic equivalent capacitance $C_1$ of the ultrasonic surgical system in the no-load state and the product $L' \times C'$ of the dynamic equivalent inductance $L'$ and the dynamic equivalent capacitance $C'$ of the ultrasonic surgical system in the on-load state are made to satisfy the relational expression (1).

According to the present disclosure, the ultrasonic surgical system further comprises a non-metallic tube arranged between the boss of the ultrasonic amplitude transformer and the support sleeve and having a friction coefficient less than or equal to 0.1.

According to the present disclosure, the non-metallic tube comprises a polytetrafluoroethylene (TFE) tube or a tetrafluoroethylene-propylene rubber (TFEP) tube.

According to the present disclosure, the outer contour of a peripheral portion of the at least one boss is configured such that the non-metallic tube is spaced apart from the support sleeve in at least one position of the peripheral portion of the at least one boss, so as to form, along the support sleeve, a through channel allowing gas and/or liquid to pass.

According to the present disclosure, the outer contour of a peripheral portion of the at least one boss is configured such that the polytetrafluoroethylene tube or tetrafluoroethylene-propylene rubber tube is spaced apart from the support sleeve in at least one position of the peripheral portion of the at least one boss, so as to form, along the support sleeve, a through channel allowing gas and/or liquid to pass. According to the present disclosure, the effective through-flow cross sectional area of the through channel is 1% to 25% of the hollow cross sectional area defined by the support sleeve, preferably 2% to 15% of the hollow cross sectional area defined by the support sleeve, and more preferably 2% to 10% of the hollow cross sectional area defined by the support sleeve, for example, 3% to 5%.

According to the present disclosure, the resonance frequency ranges between 20 kHz and 100 kHz, preferably between 30 kHz and 60 kHz, and more preferably between 43 kHz and 45 kHz or between 52 kHz and 54 kHz, and may be any specific value, for example, 43.5 kHz, 44 kHz, 44.5 kHz, 52.5 kHz, 53 kHz, 53.5 kHz, etc., within the numerical ranges.

Brief Description of the Drawings

The technical solutions of the present disclosure will be further described below in detail in conjunction with the accompanying drawings and the detailed description of embodiments, in which:

Fig. 1            is a schematic diagram of an ultrasonic surgical system according to one embodiment of the present disclosure;

Figs. 2A-2F       are respectively a front view, a left view, a right view, a top view, a bottom view, and a perspective view of a terminal end portion of the ultrasonic surgical system shown in Fig. 1;

Fig. 2G           is an exploded perspective view of the terminal end portion, shown in Figs. 2A-2F, of the ultrasonic surgical system;

Figs. 3A to 3F    are respectively a front view, a left view, a right view, a top view, a bottom view, and a perspective view of the terminal end portion, in a clamping state, of the ultrasonic surgical system shown in Fig. 1;

Figs. 3G-3I       are cutaway views respectively taken along lines A-A, B-B, and C-C in Fig. 3A;

Fig. 3J           is an exploded perspective view of the terminal end portion, in the clamping state, shown in Figs. 3A-3F, of the ultrasonic surgical system;

Fig. 4A           is a perspective view of an ultrasonic amplitude transformer of the ultrasonic surgical system according to one embodiment of the present disclosure;

Fig. 4B           is an partially enlarged view of the portion, containing the working part, of the ultrasonic amplitude transformer shown in Fig. 4A;

Figs. 4C-4E       are variations of the working part of the ultrasonic amplitude transformer.

Fig. 5A           shows an equivalent circuit diagram of the ultrasonic surgical system according to the present disclosure in a no-load state (the working part does not come into contact with other objects, i.e., does not perform incision and/or coagulation for a target tissue), and Fig. 5B shows an equivalent circuit diagram of the ultrasonic surgical system in an on-load state.

Fig. 6            shows a tendency chart of the change, varying as clamping pressure, of the resonance frequency offset of the ultrasonic surgical system according to the present disclosure when clamping two types of tissue.

Fig. 7            shows a tendency chart of the change, varying as temperature, of the resonance frequency of the ultrasonic surgical system according to the present disclosure in an on-load operation.

Detailed Description of Embodiments

The specific details of the technical solutions of the present disclosure will be described in detail below, such that those of ordinary skill in the art could have thorough understanding of the overall structure, functions, manufacturing, and use of the embodiments described in the specification and illustrated in the accompanying drawings. However, those of ordinary skill in the art should understand that the technical solutions of the present disclosure can be implemented without these specific details. The structures, elements, and operations thereof well known to those skilled in the art have not been described in detail to avoid obscuring the technical solutions described and claimed in the specification. Those of ordinary skill in the art should understand that the technical solutions described and illustrated in this specification are not limited examples, and the specific structure and functional details disclosed herein are representative and exemplary. Variations and changes of these technical solutions can be made without departing from the scope of protection as defined in the claims.

The expressions "various embodiments", "some embodiments", "one embodiment" or "an embodiment", etc. mentioned in this specification means that the specific features, structures or characteristics described in conjunction with the embodiment can also be included in at least one further embodiment. Thus, the expression "in various embodiments", "in some embodiments" or "in one embodiment", etc. mentioned in this specification do not necessarily all refer to the same embodiment. Furthermore, the specific features, structures or characteristics of the various embodiments can be combined in any suitable manner to form a novel technical solution. Thus, all or some of the specific features, structures or characteristics shown or described in conjunction with one embodiment can be combined with the features, structures or characteristics of one or more of other embodiments to form a novel technical solution, without limitation.

For clarity, the terms "proximal side" and "distal side" in this specification are defined with respect to an operator (such as a surgeon) holding an ultrasonic surgical system having a distal end effector assembly. The term "proximal side" refers to the position of an element closer to the operator, and the term "distal side" refers to the position of an element closer to the end effector assembly of the ultrasonic surgical system and farther away from the operator.

To facilitate understanding, three working states of the ultrasonic surgical system according to the present disclosure are first defined:

1. a no-load state, i.e., no clamping pressure being applied to tissue;
2. an on-load state, i.e., only applying clamping pressure to the tissue, or applying clamping pressure to the tissue and outputting power less than or equal to 1 watt; and
3. an on-load operation state, i.e., applying clamping pressure to the tissue and outputting power for incision and/or coagulation for the tissue.

It should be understood that the working states of the ultrasonic surgical system according to the present disclosure are not limited to the three working states described above, but may also include other possible working states.

In order to overcome or mitigate one or more of the defects described above in the prior art, the applicant has found through research that, for an expected load of the ultrasonic surgical system 10, i.e., for the expected type of the target tissue to be incised/coagulated and the desired magnitude of clamping pressure, the overall structure and/or the components of the ultrasonic surgical system 10 can be designed such that the resonance frequency of the ultrasonic surgical system 10 will not change with the change of the expected load or just change a little with the change of the expected load. Here, "not changing with the change of the expected load or just changing a little with the change of the expected load" refers to the resonance frequency $f_s$ of the ultrasonic surgical system in a no-load state and the resonance frequency $f_s'$ thereof in an on-load state satisfying the following relational expression (2):

$$\left| 1 - \frac{f_s'}{f_s} \right| = \alpha \qquad (2)$$

Wherein, $\alpha$ is less than or equal to 0.1%, and preferably $\alpha$ is less than or equal to 0.05%

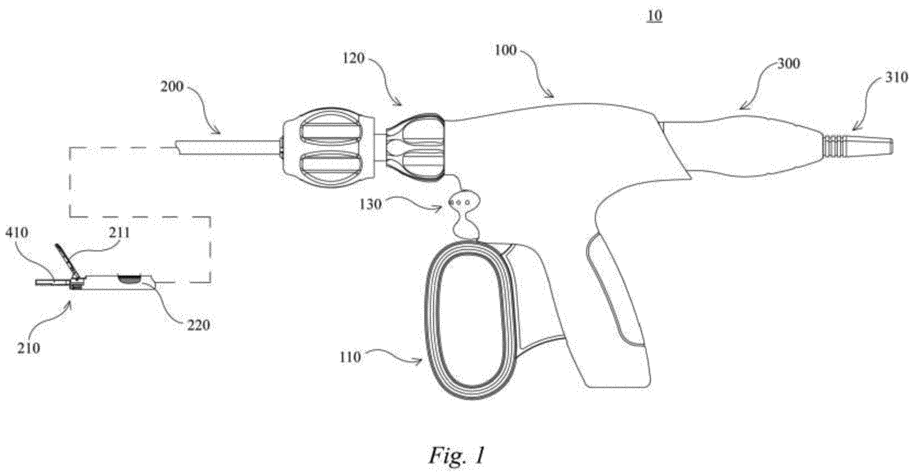

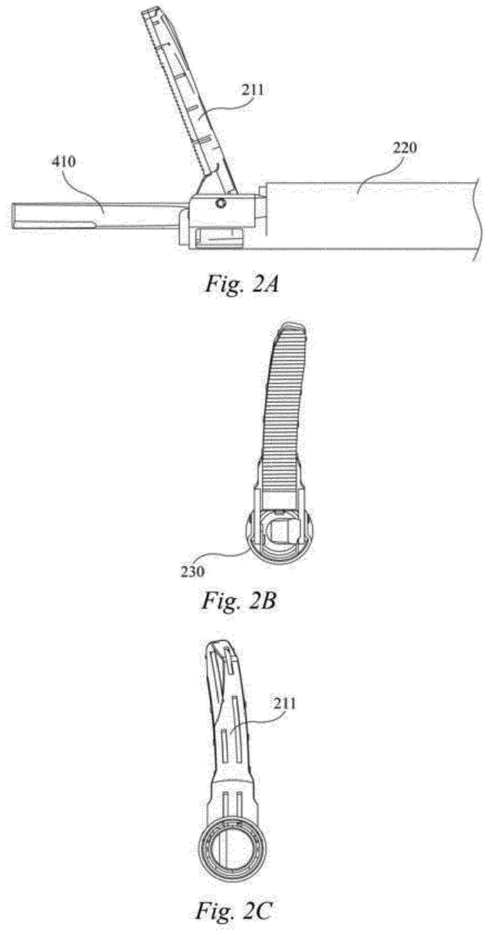

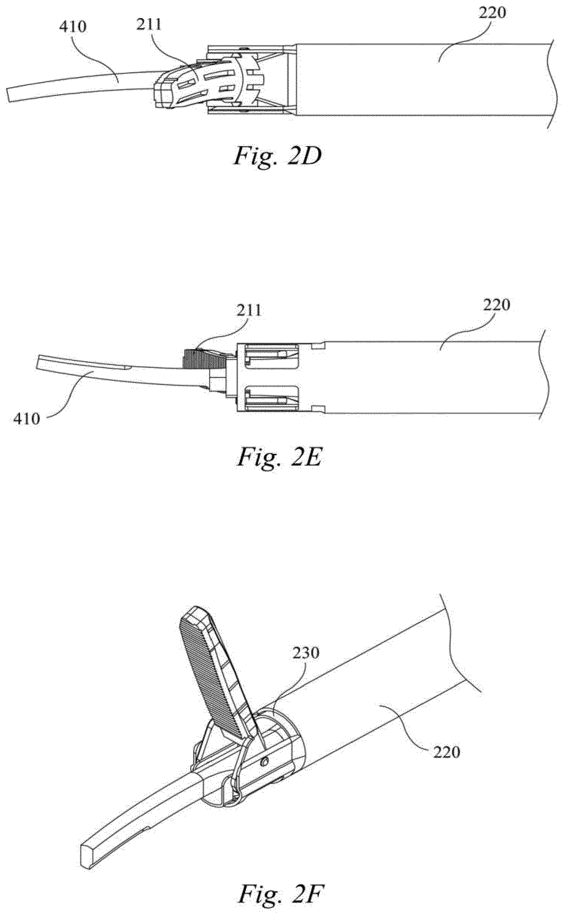

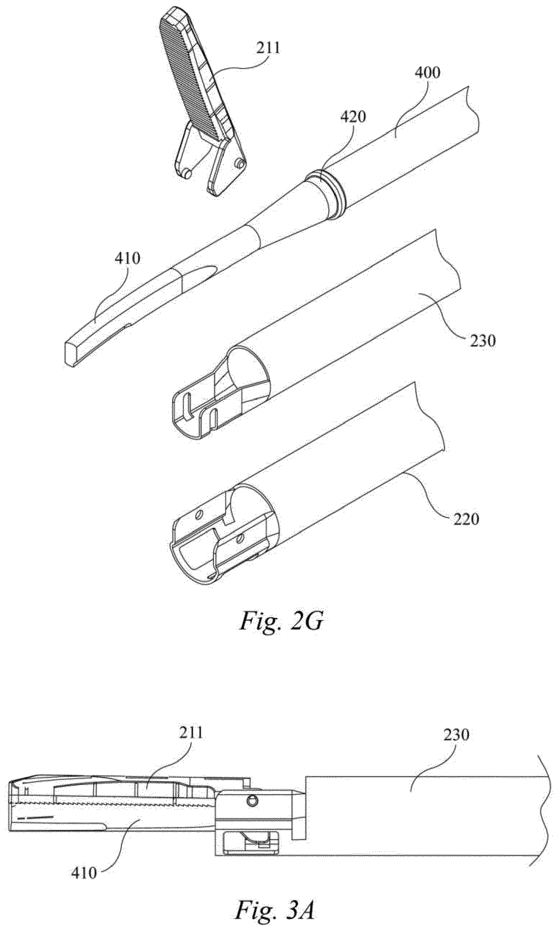

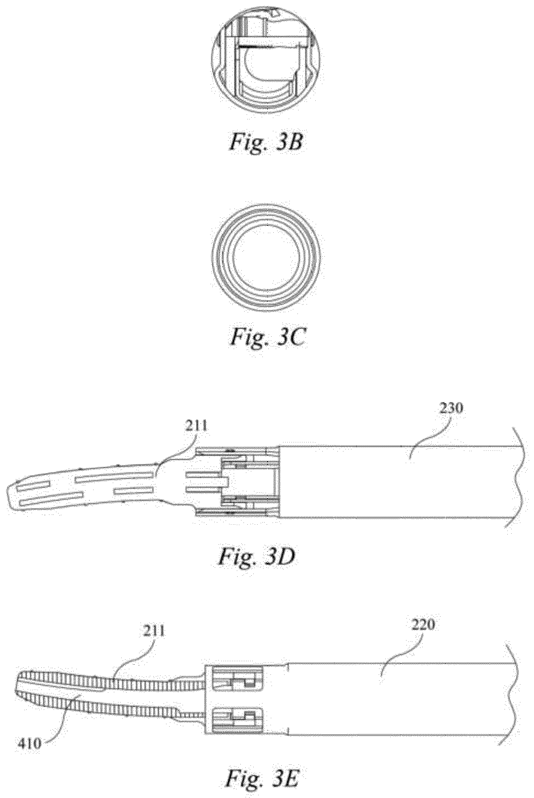

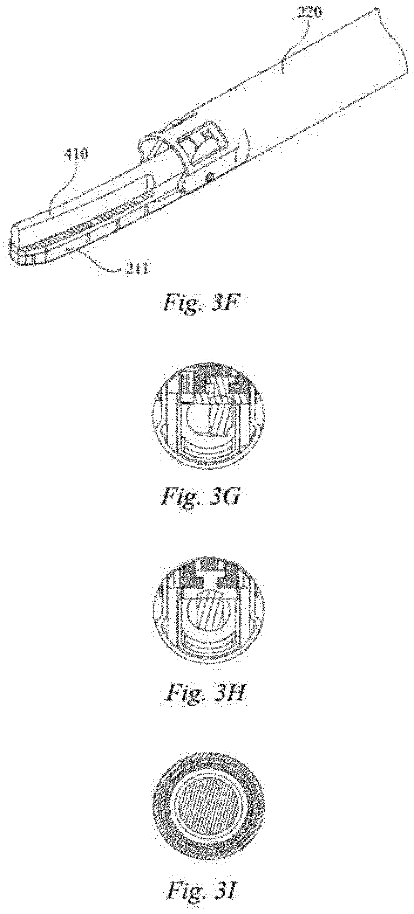

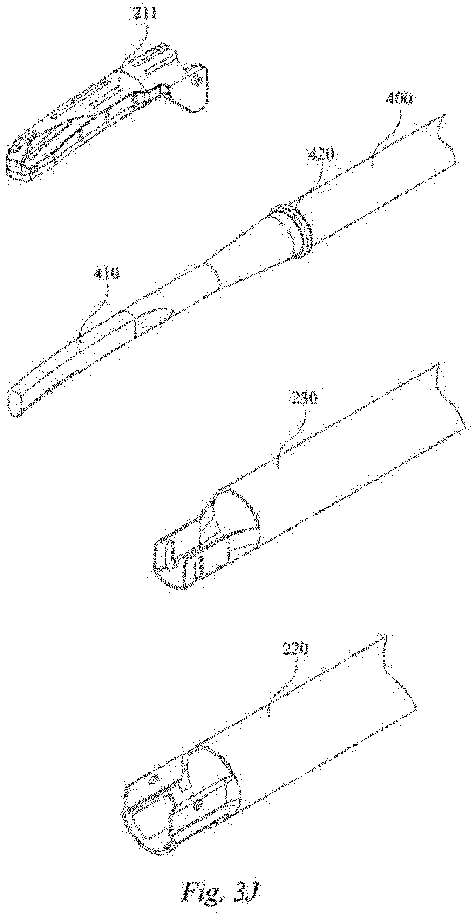

Fig. 1 shows a side view of one embodiment of the ultrasonic surgical system 10 according to the present disclosure having the above feature (i.e., the resonance frequency of the ultrasonic surgical system 10 will not change with the change of the expected load or just change a little with the change of the expected load). In the exemplary embodiment, the ultrasonic surgical system 10 can be used in a variety of surgical procedures, including endoscopic operations or conventional open surgical operations. In one exemplary embodiment, the ultrasonic surgical system 10 comprises a handle assembly 100, an elongate shaft assembly 200, an ultrasonic transducer 300, and an ultrasonic amplitude transformer 400. The handle assembly 100 comprises a trigger assembly 110, a distal rotation assembly 120, and a switch assembly 130. The elongate shaft assembly 200 comprises an end effector assembly 210. The end effector assembly 210 comprises a clamping element 211. The clamping element 211 is pivotable so as to be in a separated configuration (see Figs. 1 and 2A-2G) or in an engaged configuration (see Figs. 3A-3J) for clamping the target tissue, relative to a working part 410 (also referred to as a blade element) at a terminal end of the ultrasonic amplitude transformer 400.

The handle assembly 100 can receive the ultrasonic transducer 300 at a proximal end. The ultrasonic transducer 300 can be mechanically and acoustically engaged to the elongate shaft assembly 200 and the ultrasonic amplitude transformer 400 so as to transfer the ultrasonic vibration generated by the ultrasonic transducer 300 to the elongate shaft assembly 200 and the ultrasonic amplitude transformer 400. The ultrasonic transducer 300 can be electrically connected to a generator (not shown) through a cable 310 at a terminal end thereof. In some cases, the generator can be, for example, combined with the handle assembly 100, and electrically connected to the ultrasonic transducer 300 through an electrical connection component in the handle assembly 100, such that the ultrasonic transducer 300 no longer needs a connection cable so as to be able to conveniently replace the ultrasonic transducer 300 and prevent the tangling of cables, caused by the generator being directly connected to the cable 310 of the ultrasonic transducer 300, and the affection to the surgical operation of the surgeon due to non-uniform drag force.

According to the present disclosure, the handle assembly 100 is configured to substantially isolate the surgeon from the vibration of acoustic assemblies of the ultrasonic transducer 300, ultrasonic amplitude transformer 400, etc. so as to prevent the affection of the vibration on the surgical operation of the surgeon.

Although the structure and construction of the generator are not described in detail here, those of ordinary skill in the art should understand that the generator can comprise several functional elements or modules. Different functional elements or modules can be configured to drive different types of surgical devices. For example, an ultrasonic generator module of the generator can drive an ultrasonic device, such as the ultrasonic transducer of the ultrasonic surgical system 10. The generator may comprise a control system integrated with the generator, and a foot switch connected to the generator through a cable. The generator may also comprise a trigger mechanism for activating a surgical instrument, such as the ultrasonic surgical system 10. The trigger mechanism may comprise a power switch and a foot switch. The generator, when activated by the foot switch, can provide energy to drive the acoustic assembly (i.e., ultrasonic transducer 300) of the ultrasonic surgical system 10. The generator can drive or excite the ultrasonic transducer 300 at any suitable resonance frequency of the acoustic assembly. An electrical signal provided to the ultrasonic transducer 300 enables the working part 410 at the terminal end of the ultrasonic amplitude transformer 400 to perform longitudinal vibration or longitudinal and torsional compound vibration in the range of, for example, about 20 kHz to 100 kHz, and the vibration generates local thermal energy and vibration energy in the clamped tissue, thereby promoting the coagulation hemostasis (such as by denaturing proteins in tissue cells) and incision. According to various embodiments, the working part 410 at the terminal end of the ultrasonic amplitude transformer 400 can vibrate at a frequency in a range between 30 kHz and 60 kHz, preferably between 43 kHz and 45 kHz or between 52 kHz and 54 kHz, for example, at 43.5 kHz, 44 kHz, 44.5 kHz, 52.5 kHz, 53 kHz, 53.5 kHz, etc.

In the embodiment of the present disclosure, as shown in Figs. 1, 2A-2G, 3A-3J, and 4A-4E, the elongate shaft assembly 200 is mechanically engaged to the handle assembly 100 and the distal rotation assembly 120 at the opposite end of the end effector assembly 210. The elongate shaft assembly 200 comprises an external tubular sheath 220 and a reciprocating tubular actuation member (support sleeve) 230 located in the external tubular sheath 220. A proximal end of the reciprocating tubular actuation member 230 is mechanically engaged to the trigger assembly 110 of the handle assembly 100 to axially move in a linear motion in the external tubular sheath 200 in response to the actuation and/or release of the trigger assembly 110 so as to actuate the clamping element 211 of the end effector assembly 210. The pivotal rotation of the trigger assembly 110 is converted into the axial reciprocating movement of the reciprocating tubular actuation member 230 by means of a series of connection rods, and the axial reciprocating movement controls the clamping element 211 of the end effector assembly 210 to switch between a separated configuration (see Figs. 1 and 2A-2G) and an engaged configuration (see Figs. 3A-3J) for clamping the target tissue, relative to the working part 410 (also referred to as the blade element) of the ultrasonic amplitude transformer 400. In the separated configuration, the clamping element 211 and the working part 410 are spaced apart from each other, and in the engaged configuration, the clamping element 211 and the working part 410 cooperate to clamp, with proper clamping pressure, the target tissue located between the clamping element 211 and the working part 410. The clamping element 211 may comprise a clamping pad (not shown) to engage the tissue located between the working part 410 and the clamping element 211. The trigger assembly 110 is released to enable the clamping element 211 to move from the engaged configuration to the separated configuration.

The structures and operation of the handle assembly 100, the elongate shaft assembly 200, and the end effector assembly 210 described above are well known to those skilled in the art and may be a handle assembly, an elongate shaft assembly, and an end effector assembly described in the Chinese Patent Applications CN 106999203 A, CN 108366827 A, CN 107205775 A, for example.

In the embodiment of the present disclosure, the ultrasonic transducer 300 comprises a defined transducer housing 310, at least two piezoelectric elements (not shown), and a fastener (not shown), the transducer housing 310 defining an opening that accommodates the at least two piezoelectric elements (not shown), and the fastener being engaged to the transducer housing 310 to close the opening and apply a compressive force to the at least two piezoelectric elements. The structure and operation of the ultrasonic transducer that can be used in the ultrasonic surgical system 10 are known to those of ordinary skill in the art and thus will not be described in detail here.

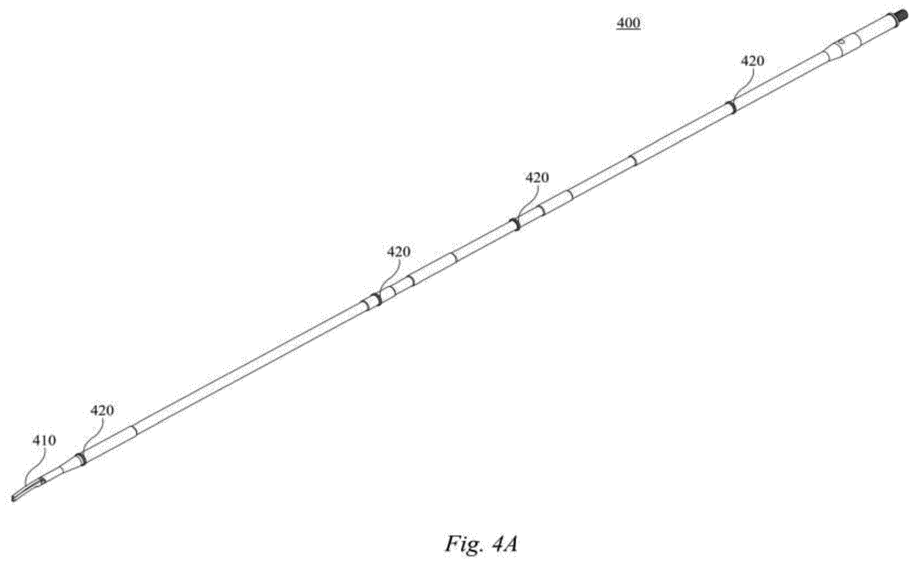

In the embodiment of the present disclosure, as shown in Figs. 1, Figs. 2A to 2G, Figs. 3A to 3J and Figs. 4A to E, the ultrasonic amplitude transformer 400 is arranged in the reciprocating tubular actuation member (support sleeve) 230. Moreover, in order to prevent the friction between the ultrasonic amplitude transformer 400, deformed by the pressure applied for clamping the target tissue during the surgical procedure, and a support mechanism (such as the reciprocating tubular actuation member 230), it is necessary to provide at least one boss 420 (four bosses 420 shown in Fig. 4A) at the position of a node (the point with the amplitude being zero) when the ultrasonic surgical system 10 vibrates at a resonance frequency such that the ultrasonic amplitude transformer 400 is supported by the reciprocating tubular actuation member 230 at the boss 420. The node position here refers to the position, where the vibration is an integer multiple of half-wavelength, on the ultrasonic amplitude transformer 400.

It should be noted that the ultrasonic amplitude transformer 400 according to the present disclosure, other than the conduction and amplification of the vibration generated by the ultrasonic transducer 300 to perform incision and/or coagulation hemostasis for the target tissue at the working part 410 by means of longitudinal vibration or longitudinal and torsional compound vibration, may also be used for conduction of electrical current to additionally cauterize the target tissue. This is very advantageous under some surgical conditions.

As one possible embodiment, the ultrasonic surgical system 10 of the present disclosure may further comprise a material with a low coefficient of friction, for example, a polytetrafluoroethylene (TFE) tube or a tetrafluoroethylene-propylene rubber (TFEP) tube, arranged between the boss 420 of the ultrasonic amplitude transformer 400 and the reciprocating tubular actuation member 230.

As one possible embodiment, the outer contour of a peripheral portion of the at least one boss 420 is configured such that the peripheral portion of the boss 420 is spaced apart from an inner surface of the reciprocating tubular actuation member 230 or, in the case where a polytetrafluoroethylene tube or tetrafluoroethylene-propylene rubber tube is provided, the polytetrafluoroethylene tube or tetrafluoroethylene-propylene rubber tube is space apart from the inner surface of the reciprocating tubular actuation member 230 in at least one position of the peripheral portion of the at least one boss 420, so as to form, along the reciprocating tubular actuation member 230, a through channel allowing gas and/or liquid to pass through. This is very advantageous in the case where the ultrasonic surgical system 10 is applied to closed surgery such as laparoscopic surgery, because gas (such as nitrogen gas) is usually injected into the surgical site in such closed surgery, with the temperature of the injected gas being much lower than the work temperature, such as 36°C, of the ultrasonic surgical system 10, especially of the ultrasonic amplitude transformer 400. By means of forming the through channel, during the surgical operation, the gas can pass through the through channel for cooling the ultrasonic surgical system 10, especially the ultrasonic amplitude transformer 400. It should be understood that the through channel, other than allowing the gas of the surgical site to pass through in a closed surgical environment, may also be used to deliver cooling gas or liquid from the outside to the surgical site, which is especially suitable for open surgery. As an example, the through channel can be formed by means of removing a part of the peripheral portion of the boss 420.

In one embodiment, since in the closed surgical environment, it is necessary to maintain the surgical site to be in a positive pressure state with the injected gas, the effective through-flow cross sectional area of the through channel is set to between 1% and 25% of the hollow cross sectional area defined by the reciprocating tubular actuation member 230, preferably between 2% and 15% of the hollow cross sectional area defined by the reciprocating tubular actuation member 230, and more preferably between 2% and 10% or between 3% and 5% of the hollow cross sectional area defined by the reciprocating tubular actuation member 230, so as to cool the ultrasonic amplitude transformer 400 and other components while maintaining the surgical site to be in a positive pressure state.

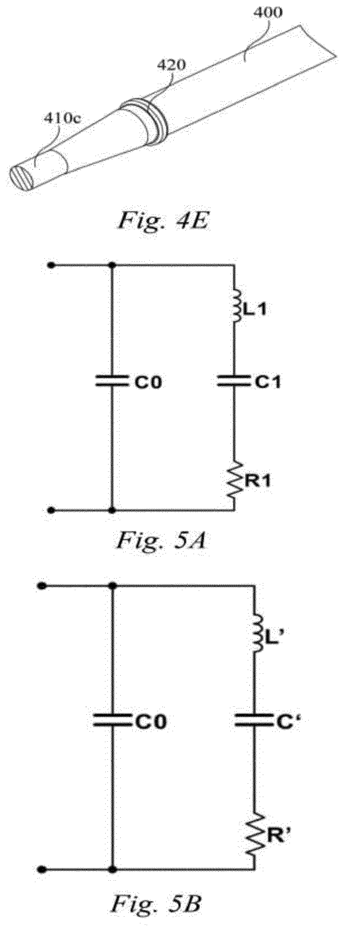

Fig. 5A shows an equivalent circuit diagram of the ultrasonic surgical system 10 according to the present disclosure in a no-load state (when the working part 410 does not come into contact with other objects, i.e., does not perform clamping, incision and/or coagulation for the target tissue), comprising a static equivalent capacitance $C_0$ formed by a piezoelectric element (such as piezoelectric ceramic), and a dynamic equivalent inductance $L_1$, a dynamic equivalent capacitance $C_1$ and a dynamic equivalent resistance $R_1$ of a mechanical vibration system. Fig. 5B shows an equivalent circuit diagram of the ultrasonic surgical system 10 in an on-load state, comprising a dynamic equivalent inductance L', a dynamic equivalent capacitance C' and a mechanical load equivalent resistance R'. According to one embodiment of the present disclosure, the ultrasonic surgical system 10 can be designed such that the product $L_1 \times C_1$ of the dynamic equivalent inductance $L_1$ and the dynamic equivalent capacitance $C_1$ of the ultrasonic surgical system in the no-load state and the product L' x C' of the dynamic equivalent inductance L' and the dynamic equivalent capacitance C' of the ultrasonic surgical system in the on-load state satisfy the following relational expression (1),

$$\left| 1 - \frac{\sqrt{L_1 \times C_1}}{\sqrt{L' \times C'}} \right| = \alpha \tag{1}$$

such that a resonance frequency $f_s$ of the ultrasonic surgical system in the no-load state and a resonance frequency $f_s'$ thereof in the on-load state satisfy the following relational expression (2):

$$\left| 1 - \frac{f_s'}{f_s} \right| = \alpha \tag{2}$$

$$f_s = \frac{1}{2\pi\sqrt{L_1 \times C_1}}, \qquad f_s' = \frac{1}{2\pi\sqrt{L' \times C'}},$$

wherein, and

$\alpha$ is less than or equal to 0.1%, and preferably $\alpha$ is less than or equal to 0.05%.

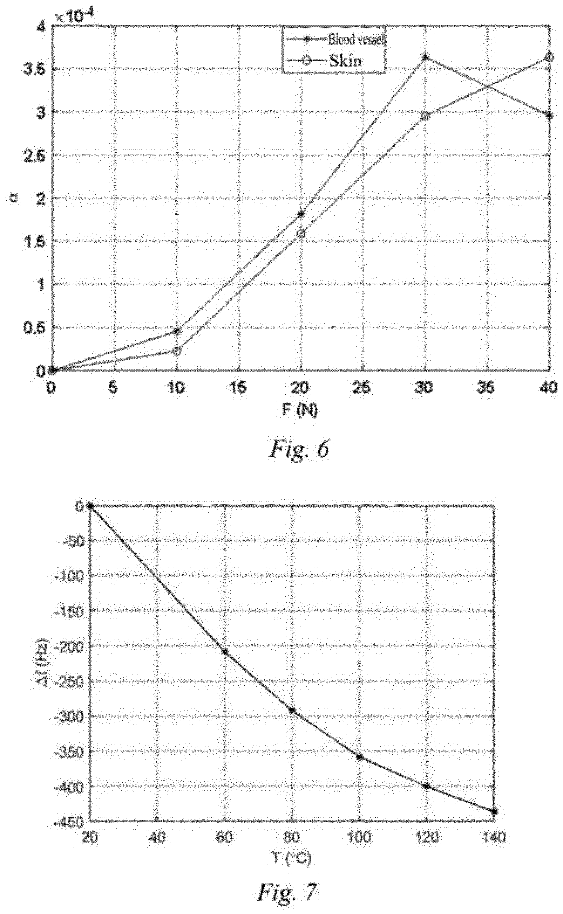

Fig. 6 shows a tendency chart of the change, with clamping pressure F, of the relative resonance frequency

$$\alpha = \left| 1 - \frac{f_s'}{f_s} \right|$$

offset of the ultrasonic surgical system according to the present disclosure when clamping a vascular tissue (Vessel) and a skin tissue (Skin) at room temperature (such as 20°C), tested using an impedance analyzer. It can be seen from Fig. 6 that $\alpha$ is less than 0.04% as the clamping pressure increases from 0 N to 40 N.

Fig. 7 shows a tendency chart of the change, with temperature of the working part, of the resonance frequency

offset $\Delta f = f_s' - f_s$ of the ultrasonic surgical system according to the present disclosure in an on-load operation state. The applicant was surprised to find that when the ultrasonic surgical system 10 has the above features (i.e., the resonance frequency of the ultrasonic surgical system in a no-load state and the resonance frequency thereof in an on-load state satisfy the relational expression (2)), the resonance frequency offset of the ultrasonic surgical system 10 and the temperature of the working part 410 of the ultrasonic surgical system 10 satisfy a one-to-one functional relationship (see Fig. 7), such that the real-time or near-real-time temperature of the working part 410 of the ultrasonic surgical system 10 can be determined only according to the resonance frequency offset of the ultrasonic surgical system 10 so as to determine the real-time or near-real-time temperature of the target tissue undergoing incision and/or coagulation.

It should be noted that various methods and devices for estimating the temperature of the working part of the ultrasonic surgical system or the target tissue undergoing incision and/or coagulation have been disclosed in the prior art, but these methods and devices have poor accuracy or severe hysteresis (i.e., being unable to realize real-time or near-real-time temperature measurement). For example, the Chinese invention patent application CN 101772325 A discloses: 1) the temperature of an ultrasonic end effector in use being roughly determined by means of measuring the resonance frequency of an ultrasonic system and correlating the change of frequency of an end effector with the temperature of the end effector; and 2) the temperature of the end effector being directly measured with a temperature sensor (such as a thermocouple, an acoustic sensor, or a thermistor type device).

For the feature 1) the temperature of an ultrasonic end effector when in use being roughly determined by means of measuring the resonance frequency of an ultrasonic system and correlating the change of frequency of an end effector with the temperature of the end effector, disclosed in the Chinese invention patent application CN 101772325 A, there is a defect in that since the resonance frequency of the ultrasonic system is greatly affected by the load (the type of the tissue to be incised and/or coagulated and/or the magnitude of clamping pressure and/or the length of the clamped target tissue), the change of resonance frequency does not have a one-to-one functional relationship or an accurate one-to-one functional relationship with the temperature of the end effector, i.e., the amount of change of a particular resonance frequency may correspond to two or more different temperatures. In contrast, the resonance frequency of the ultrasonic surgical system 10 according to the present disclosure will not change with the change of load or just change a little with the change of load, such that the resonance frequency offset of the ultrasonic surgical system 10 of the present disclosure and the temperature of the working part 410 of the ultrasonic surgical system 10 satisfy the one-to-one functional relationship, i.e., the temperature of the working part 410 can be determined accurately and in near real time only according to the resonance frequency offset. The accuracy and timeliness of the determined temperature are significantly improved compared with the prior art.

For the feature 2) the temperature of the end effector being directly measured with a temperature sensor (such as a thermocouple, an acoustic sensor, or a thermistor type device) disclosed in the Chinese invention patent application CN 101772325 A, there are defects in that a temperature sensor is additionally provided, which makes the structure more complex and increases the manufacturing cost, and due to the limitation of the working principle of the temperature sensor (such as the thermocouple, the acoustic sensor or the thermistor type device), the measured temperature has severe hysteresis and cannot reflect the actual condition of the surgical operation in time.

The applicant also found that when the resonance frequency of the ultrasonic surgical system 10 will not change with load or only have a negligible change with load, the ultrasonic surgical system 10 can be significantly simplified in terms of the mechanical design, the manufacturing accuracy and the assembly accuracy of components, thereby significantly reducing the manufacturing cost of the ultrasonic surgical system 10. Since the resonance frequency of the designed ultrasonic surgical system 10 is little affected by the change of load, a simpler support or vibration isolation mechanical structure can be used. For example, a hard support can be formed between the ultrasonic amplitude transformer and the reciprocating tubular actuation member (support sleeve) 230 in a structural mode of a boss 420, so as to avoid formation of a soft support between the ultrasonic amplitude transformer and the support sleeve formed by an elastic member being machined on the boss by means of bonding, injection molding and other processes, thereby reducing the machining cost of the ultrasonic surgical system 10. As described above, a polytetrafluoroethylene tube may be provided between the boss 420 and the reciprocating tubular actuation member (support sleeve) 230.

As well known to those of ordinary skill in the art, not all components of the ultrasonic surgical system will wear at the same rate. For example, for the ultrasonic surgical system, the ultrasonic amplitude transformer may wear and become unavailable faster than other components of the ultrasonic surgical system, and the elongate shaft assembly may have longer service life than the components comprising the end effector, and the ultrasonic transducer may have longer service life. In the prior art, although the soft support between the ultrasonic amplitude transformer and the support sleeve formed by an elastic member being machined on the boss by means of bonding, injection molding and other processes can achieve a good effect of isolating unexpected vibration, the elastic member becomes unusable before the ultrasonic amplitude transformer reaches its service life since the elastic member cannot be removed and replaced from the ultrasonic amplitude transformer and the elastic member cannot withstand high-temperature sterilization for enough times due to the material limitation, as a result, and accordingly the service life of the ultrasonic amplitude transformer is further shortened because of the elastic member, causing serious waste. In contrast, since the resonance frequency of the ultrasonic surgical system 10 will not change with load or only have a negligible change with load, no unexpected violent vibration will be generated at the position of the boss 420, such that it is possible to use a hard support engagement method between the ultrasonic amplitude transformer 400 and the reciprocating tubular actuation member 230, and in one embodiment according to the present disclosure, a detachable polytetrafluoroethylene tube can be provided between the boss 420 and the reciprocating tubular actuation member 230. The polytetrafluoroethylene tube has the technical advantages in that the polytetrafluoroethylene tube is more resistant to high-temperature sterilization, has longer service life, and can be conveniently and independently replaced by a surgeon or other auxiliary personnel (i.e., only the polytetrafluoroethylene tube is replaced without replacing the ultrasonic amplitude transformer), thereby reducing the overall usage cost of the ultrasonic surgical system 10.

In one feasible embodiment, the product $L_1 \times C_1$ of the dynamic equivalent inductance $L_1$ and the dynamic equivalent capacitance $C_1$ of the ultrasonic surgical system 10 in a no-load state and the product $L' \times C'$ of the dynamic equivalent inductance $L'$ and the dynamic equivalent capacitance $C'$ of the ultrasonic surgical system in an on-load state satisfy the relational expression (1) within an expected tissue clamping pressure range of the working part 410 of the ultrasonic surgical system 10, such that the resonance frequency of the ultrasonic surgical system 10 in a no-load state and the resonance frequency thereof in an on-load state satisfy the relational expression (2) within the expected tissue clamping pressure range. The expected tissue clamping pressure range is 1 N to 40 N, preferably, the expected tissue clamping pressure range is 5 N to 36 N, and more preferably, the expected tissue clamping pressure range is 10 N to 36 N.

The overall structure and/or the components of the ultrasonic surgical system 10 can be designed, such that the resonance frequency of the ultrasonic surgical system 10 will not change with the change of the expected load or just change a little with the change of the expected load, by means of adjusting and designing the following parameters of the ultrasonic surgical system 10:

1. Any one or a combination of any two or more of the following parameters a, b, and c of the ultrasonic transducer 300: a: the number of the piezoelectric elements; b: the diameter of the piezoelectric element; and c: a compressive force applied to the piezoelectric element.

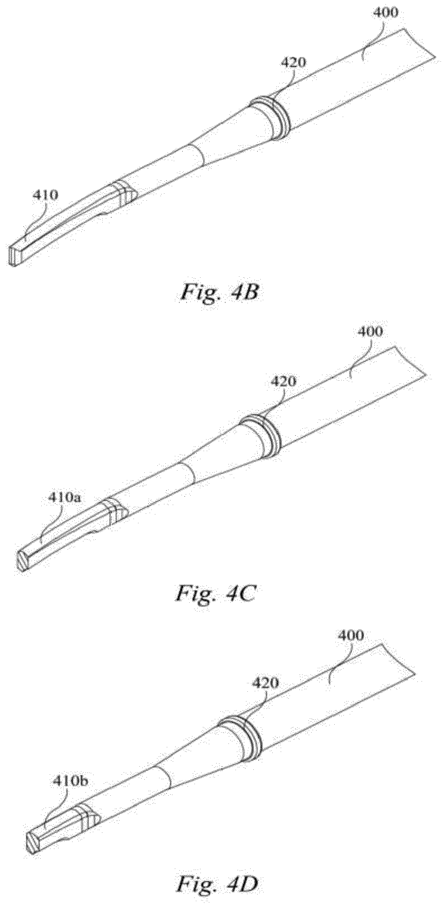

2. Any one or a combination of any two or more of the length of the ultrasonic amplitude transformer 400, the change of cross section of the ultrasonic amplitude transformer 400 along the longitudinal axis, and the change of cross section of the working part 410 along the longitudinal axis. As shown in Fig. 4A, the cross section of the ultrasonic amplitude transformer 400 has multiple segments with different diameters along the longitudinal axis thereof such that the cross section of the ultrasonic amplitude transformer 400 varies along the longitudinal axis. In addition, Figs. 4B, 4C, 4D and 4E show different designs of the working part 410 (i.e., the cross section of the working part 410 varies along the longitudinal axis in different manners). It should be understood that although Fig. 4A shows that the cross section of the ultrasonic amplitude transformer 400 discontinuously varies along the longitudinal axis thereof (i.e., having multiple segments with different diameters along the longitudinal axis thereof), the cross section of the ultrasonic amplitude transformer 400 may continuously vary along the longitudinal axis thereof.

3. The number of the boss 420 and/or the position of the boss 420 of the ultrasonic amplitude transformer 400.

Since the resonance frequency of the ultrasonic surgical system 10 according to the present disclosure will not change with the change of the expected load or just change a little with the change of the expected load, the requirements of manufacturing and assembly accuracy for the components of the ultrasonic surgical system 10 are significantly reduced, especially significantly reducing the requirements of manufacturing and assembly accuracy for the components associated with the application of clamping pressure to the target tissue. This is because even with the reduced manufacturing and assembly accuracy and/or increased tolerance of the components associated with the application of clamping pressure to the target tissue, the resulting difference between expected clamping pressure and the applied clamping pressure will not affect or only have negligible affection on the resonance frequency of the ultrasonic surgical system 10 according to the present disclosure. In addition, the affection of the difference between the expected clamping pressure or the expected applied pressure and the applied clamping pressure on the resonance frequency of the ultrasonic surgical system 10 according to the present disclosure may not be considered, the control over the ultrasonic surgical system 10 is further significantly simplified.

It should be understood by those of ordinary skill in the art that the above adjustable and designable parameters of the ultrasonic surgical system 10 are not exhaustive, and by means of adjusting and designing other parameters of the ultrasonic surgical system 10, the overall structure and/or the components of the ultrasonic surgical system 10 can be designed, such that the resonance frequency of the ultrasonic surgical system 10 will not change with the change of the expected load or just change a little with the change of the expected load.

In one feasible embodiment, the resonance frequency ranges between 20 kHz and 100 kHz, preferably, between 30 kHz and 60 kHz, and more preferably, between 43 kHz and 45 kHz or between 52 kHz and 54 kHz, and may be any specific value, for example, 43.5 kHz, 44 kHz, 44.5 kHz, 52.5 kHz, 53 kHz, 53.5 kHz, etc., within the above numerical ranges.

Those skilled in the art would have appreciated that the present disclosure may have various other embodiments, without departing from the spirit and essence of the present disclosure, a person skilled in the art would have been able to make various changes and modifications in accordance with the present disclosure, and these corresponding changes and modifications all fall within the scope of protection of the appended claims of the present disclosure.

**Claims**

1. An ultrasonic surgical system (10), comprising:

   an ultrasonic transducer (300) adapted to convert an alternating current signal into vibration; and
   an ultrasonic amplitude transformer (400) adapted to amplify the amplitude of the vibration generated by the ultrasonic transducer (300) so as to drive a working part (410) located at a terminal end of the ultrasonic amplitude transformer (400) to perform incision and/or coagulation operation for a target tissue;
   wherein the product $L_1 \times C_1$ of a dynamic equivalent inductance $L_1$ and a dynamic equivalent capacitance $C_1$ of the ultrasonic surgical system (10) in a no-load state and the product $L' \times C'$ of a dynamic equivalent inductance $L'$ and a dynamic equivalent capacitance $C'$ of the ultrasonic surgical system (10) in an on-load state satisfy the following relational expression (1),

$$\left| 1 - \frac{\sqrt{L_1 \times C_1}}{\sqrt{L' \times C'}} \right| = \alpha \tag{1}$$

such that a resonance frequency $f_s$ of the ultrasonic surgical system (10) in the no-load state and a resonance frequency $f_s'$ thereof in the on-load state satisfy the following relational expression (2):

$$\left| 1 - \frac{f_s'}{f_s} \right| = \alpha \tag{2}$$

wherein, $f_s = \frac{1}{2\pi\sqrt{L_1 \times C_1}}$, $f_s' = \frac{1}{2\pi\sqrt{L' \times C'}}$, and
$\alpha$ is less than or equal to 0.1%, and preferably $\alpha$ is less than or equal to 0.05%.

2. The ultrasonic surgical system (10) according to claim 1, wherein the product $L_1$ x $C_1$ of the dynamic equivalent inductance $L_1$ and the dynamic equivalent capacitance $C_1$ of the ultrasonic surgical system (10) in the no-load state and the product L' x C' of the dynamic equivalent inductance L' and the dynamic equivalent capacitance C' of the ultrasonic surgical system (10) in the on-load state satisfy the relational expression (1) within an expected tissue clamping pressure range of the working part (410) of the ultrasonic surgical system (10), such that the resonance frequency $f_s$ of the ultrasonic surgical system (10) in the no-load state and the resonance frequency $f_s'$ thereof in the on-load state satisfy the relational expression (2) within the expected tissue clamping pressure range.

3. The ultrasonic surgical system (10) according to claim 2, wherein the expected tissue clamping pressure range is 1 N to 40 N, preferably 5 N to 36 N, and more preferably 10 N to 36 N.

4. The ultrasonic surgical system (10) according to any one of claims 1 to 3, wherein the target tissue comprises one or more types of soft tissue.

5. The ultrasonic surgical system (10) according to any one of claims 1 to 4, wherein during on-load operation, the resonance frequency offset of the ultrasonic surgical system (10) and the temperatures of the working part (410) of the ultrasonic surgical system (10) satisfy a one-to-one functional relationship, such that the temperature of the working part (410) of the ultrasonic surgical system (10) can be determined only according to the resonance frequency offset of the ultrasonic surgical system (10).

6. The ultrasonic surgical system (10) according to any one of claims 1 to 5, wherein the transducer comprises:

   a transducer housing (310) defining an opening;
   at least two piezoelectric elements arranged in the opening, and
   a fastener engaged with the transducer housing (310) to close the opening and apply a compressive force to the at least two piezoelectric elements such that, by means of adjusting any one or a combination of any two or more of the following parameters a, b and c of the transducer, the product $L_1$ x $C_1$ of the dynamic equivalent inductance $L_1$ and the dynamic equivalent capacitance $C_1$ of the ultrasonic surgical system (10) in the no-load state and the product L' x C' of the dynamic equivalent inductance L' and the dynamic equivalent capacitance C' of the ultrasonic surgical system (10) in the on-load state satisfy the relational expression (1):

      a: the number of the piezoelectric elements;
      b: the diameter of the piezoelectric element; and
      c: a compressive force applied to the piezoelectric element.

7. The ultrasonic surgical system (10) according to any one of claims 1 to 6, wherein any one or a combination of any two or more of the material, the length, the change in cross section along a longitudinal axis, and a change in cross section of the working part (410) along the longitudinal axis of the ultrasonic transducer (300) are adjusted such that the product $L_1$ x $C_1$ of the dynamic equivalent inductance $L_1$ and the dynamic equivalent capacitance $C_1$ of the ultrasonic surgical system (10) in the no-load state and the product L' x C' of the dynamic equivalent inductance L' and the dynamic equivalent capacitance C' of the ultrasonic surgical system (10) in the on-load state satisfy the relational expression (1).

8. The ultrasonic surgical system (10) according to any one of claims 1 to 7, further comprising a support sleeve, wherein the ultrasonic amplitude transformer (400) is arranged in the support sleeve, the ultrasonic amplitude transformer (400) is provided with at least one boss (420) at a node position, the ultrasonic amplitude transformer (400) is supported by the support sleeve at the at least one boss (420), and the number of the bosses (420) and/or the position of the bosses (420) of the ultrasonic amplitude transformer (400) is adjusted such that the product $L_1$ x $C_1$ of the dynamic equivalent inductance $L_1$ and the dynamic equivalent capacitance $C_1$ of the ultrasonic surgical system (10) in the no-load state and the product L' x C' of the dynamic equivalent inductance L' and the dynamic equivalent capacitance C' of the ultrasonic surgical system (10) in the on-load state satisfy the relational expression (1).

9. The ultrasonic surgical system (10) according to claim 8, further comprising a non-metallic tube arranged between the boss (420) of the ultrasonic amplitude transformer (400) and the support sleeve and having a friction coefficient less than or equal to 0.1.

10. The ultrasonic surgical system (10) according to claim 9, wherein the non-metallic tube comprises a polytetrafluoroethylene (TFE) tube or a tetrafluoroethylene-propylene rubber (TFEP) tube.

11. The ultrasonic surgical system (10) according to claim 9 or claim 10, wherein the outer contour of a peripheral portion of the at least one boss (420) is configured such that the non-metallic tube is spaced apart from the support sleeve in at least one position of the peripheral portion of the at least one boss (420), so as to form, along the support sleeve, a through channel allowing gas and/or liquid to pass.

12. The ultrasonic surgical system (10) according to claim 11, wherein the effective through-flow cross sectional area of the through channel is 1% to 25% of the hollow cross sectional area defined by the support sleeve.

13. The ultrasonic surgical system (10) according to any one of claims 1 to 12, wherein the resonance frequency ranges between 20 kHz and 100 kHz.

14. The ultrasonic surgical system (10) according to claim 12, wherein the effective through-flow cross sectional area of the through channel is 2% to 15% of the hollow cross sectional area defined by the support sleeve, and preferably is 2% to 10% of the hollow cross sectional area defined by the support sleeve.

15. The ultrasonic surgical system (10) according to claim 13, wherein the resonance frequency ranges between 30 kHz and 60 kHz, and preferably ranges between 43 kHz and 45 kHz or between 52 kHz and 54 kHz.

*Fig. 1*

EP 3 868 315 A1

*Fig. 2A*

*Fig. 2B*

*Fig. 2C*

15

410    211                                                    220

*Fig. 2D*

211                                              220

410

*Fig. 2E*

230

220

*Fig. 2F*

*Fig. 2G*

*Fig. 3A*

*Fig. 3B*

*Fig. 3C*

*Fig. 3D*

*Fig. 3E*

Fig. 3F

Fig. 3G

Fig. 3H

Fig. 3I

211

400

420

410

230

220

*Fig. 3J*

Fig. 4A

Fig. 4B

Fig. 4C

Fig. 4D

*Fig. 4E*

*Fig. 5A*

*Fig. 5B*

*Fig. 6*

*Fig. 7*

Europäisches
Patentamt

European
Patent Office

Office européen
des brevets

# EUROPEAN SEARCH REPORT

Application Number

EP 21 15 4898

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br>A | US 2016/374708 A1 (WIENER EITAN T [US] ET AL) 29 December 2016 (2016-12-29)<br>* paragraphs [0043], [0045], [0047], [0053], [0062] - [0064]; figures 1,3,5-9 * | 1-5,7-9, 13,15<br>6,10-12, 14 | INV.<br>A61B17/32 |
| X<br>A | US 6 159 176 A (BROADWIN ALAN [US] ET AL) 12 December 2000 (2000-12-12)<br>* column 1, paragraph 23-27 *<br>* column 4, line 14 - column 5, line 50; figures 1-5 * | 1-5,7-14<br>6,15 | |
| X<br>A | US 2019/231381 A1 (COWLEY MATTHEW S [US]) 1 August 2019 (2019-08-01)<br>* paragraphs [0030], [0035], [0039] - [0044]; claim 7; figures 1-6 * | 1-7<br>8-15 | |
| X<br>A | US 2008/234708 A1 (HOUSER KEVIN L [US] ET AL) 25 September 2008 (2008-09-25)<br>* paragraphs [0071], [0073] - [0075], [0096]; figures 2, 6,37 * | 1-5,7,8, 13,15<br>6,9-12, 14 | |
| X<br>A | US 2012/265196 A1 (TURNER DOUGLAS J [US] ET AL) 18 October 2012 (2012-10-18)<br>* paragraphs [0126], [0127], [0135], [0136], [0147], [0154], [0155], [0156]; figures 2-3,9,10 * | 1-5,7, 13,15<br>6,8-12, 14 | TECHNICAL FIELDS SEARCHED (IPC)<br><br>A61B |
| A | YING C ET AL: "Effects of different tissue loads on high power ultrasonic surgery scalpel",<br>ULTRASOUND IN MEDICINE AND BIOLOGY, NEW YORK, NY, US,<br>vol. 32, no. 3, 1 March 2006 (2006-03-01), pages 415-420, XP027881158,<br>ISSN: 0301-5629<br>[retrieved on 2006-03-01]<br>* abstract; figure 1; table 1 * | 1-15 | |

-/--

The present search report has been drawn up for all claims

| Place of search | Date of completion of the search | Examiner |
|---|---|---|
| The Hague | 23 June 2021 | van Poelgeest, A |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

**EUROPEAN SEARCH REPORT**

Application Number

EP 21 15 4898

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2019/274707 A1 (SAWHNEY AMRITA S [US] ET AL) 12 September 2019 (2019-09-12)<br>* paragraph [0271] *<br>----- | 5 | |

TECHNICAL FIELDS
SEARCHED      (IPC)

The present search report has been drawn up for all claims

| Place of search | Date of completion of the search | Examiner |
|---|---|---|
| The Hague | 23 June 2021 | van Poelgeest, A |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

&  : member of the same patent family, corresponding
document

EPO FORM 1503 03.82 (P04C01)

page 2 of 2

## ANNEX TO THE EUROPEAN SEARCH REPORT
## ON EUROPEAN PATENT APPLICATION NO.

EP 21 15 4898

This annex lists the patent family members relating to the patent documents cited in the above-mentioned European search report.
The members are as contained in the European Patent Office EDP file on
The European Patent Office is in no way liable for these particulars which are merely given for the purpose of information.

23-06-2021

| Patent document cited in search report | | Publication date | Patent family member(s) | | Publication date |
|---|---|---|---|---|---|
| US 2016374708 | A1 | 29-12-2016 | AU | 2008282147 A1 | 05-02-2009 |
| | | | CA | 2694946 A1 | 05-02-2009 |
| | | | CN | 101772327 A | 07-07-2010 |
| | | | EP | 2190363 A2 | 02-06-2010 |
| | | | EP | 2514375 A2 | 24-10-2012 |
| | | | EP | 2514376 A2 | 24-10-2012 |
| | | | EP | 2514377 A2 | 24-10-2012 |
| | | | JP | 6067208 B2 | 25-01-2017 |
| | | | JP | 2010535089 A | 18-11-2010 |
| | | | PL | 2514377 T3 | 02-11-2020 |
| | | | US | 2009036912 A1 | 05-02-2009 |
| | | | US | 2013226208 A1 | 29-08-2013 |
| | | | US | 2016374708 A1 | 29-12-2016 |
| | | | US | 2020015798 A1 | 16-01-2020 |
| | | | WO | 2009018406 A2 | 05-02-2009 |
| US 6159176 | A | 12-12-2000 | AU | 1817599 A | 28-06-1999 |
| | | | US | 6159176 A | 12-12-2000 |
| | | | WO | 9929241 A1 | 17-06-1999 |
| US 2019231381 | A1 | 01-08-2019 | NONE | | |
| US 2008234708 | A1 | 25-09-2008 | AU | 2008231088 A1 | 02-10-2008 |
| | | | CA | 2682225 A1 | 02-10-2008 |
| | | | CN | 101674781 A | 17-03-2010 |
| | | | EP | 2131761 A2 | 16-12-2009 |
| | | | JP | 2010522032 A | 01-07-2010 |
| | | | US | 2008234708 A1 | 25-09-2008 |
| | | | US | 2012259353 A1 | 11-10-2012 |
| | | | US | 2015045819 A1 | 12-02-2015 |
| | | | US | 2018168680 A1 | 21-06-2018 |
| | | | WO | 2008118707 A2 | 02-10-2008 |
| US 2012265196 | A1 | 18-10-2012 | AU | 2013249506 A1 | 16-10-2014 |
| | | | BR | 112014025700 A2 | 20-06-2017 |
| | | | CA | 2870699 A1 | 24-10-2013 |
| | | | CN | 104363849 A | 18-02-2015 |
| | | | EP | 2840995 A2 | 04-03-2015 |
| | | | JP | 6301312 B2 | 28-03-2018 |
| | | | JP | 2015515343 A | 28-05-2015 |
| | | | KR | 20150003309 A | 08-01-2015 |
| | | | MX | 342019 B | 08-09-2016 |
| | | | RU | 2014145823 A | 10-06-2016 |
| | | | US | 2012265196 A1 | 18-10-2012 |
| | | | US | 2016089533 A1 | 31-03-2016 |
| | | | WO | 2013158537 A2 | 24-10-2013 |

EPO FORM P0459

For more details about this annex : see Official Journal of the European Patent Office, No. 12/82

page 1 of 3

EP 3 868 315 A1

**ANNEX TO THE EUROPEAN SEARCH REPORT
ON EUROPEAN PATENT APPLICATION NO.**

EP 21 15 4898

This annex lists the patent family members relating to the patent documents cited in  the above-mentioned European search report.
The members are as contained in the European Patent Office EDP file on
The European Patent Office is in no way liable for these particulars which are merely given for the purpose of information.

23-06-2021

| Patent document cited in search report | Publication date | Patent family member(s) | Publication date |
|---|---|---|---|
| US 2019274707 A1 | 12-09-2019 | BR 112020017632 A2 | 22-12-2020 |
| | | BR 112020017666 A2 | 22-12-2020 |
| | | BR 112020017740 A2 | 22-12-2020 |
| | | BR 112020017815 A2 | 22-12-2020 |
| | | BR 112020017868 A2 | 22-12-2020 |
| | | BR 112020017876 A2 | 22-12-2020 |
| | | BR 112020017882 A2 | 22-12-2020 |
| | | BR 112020017952 A2 | 22-12-2020 |
| | | BR 112020017968 A2 | 22-12-2020 |
| | | BR 112020018056 A2 | 22-12-2020 |
| | | BR 112020018109 A2 | 22-12-2020 |
| | | CN 111818862 A | 23-10-2020 |
| | | CN 111818863 A | 23-10-2020 |
| | | CN 111818865 A | 23-10-2020 |
| | | CN 111818866 A | 23-10-2020 |
| | | CN 111818868 A | 23-10-2020 |
| | | CN 111836590 A | 27-10-2020 |
| | | CN 111836591 A | 27-10-2020 |
| | | CN 111836592 A | 27-10-2020 |
| | | CN 111867500 A | 30-10-2020 |
| | | CN 111885974 A | 03-11-2020 |
| | | CN 112074244 A | 11-12-2020 |
| | | JP 2021514786 A | 17-06-2021 |
| | | JP 2021514788 A | 17-06-2021 |
| | | JP 2021514789 A | 17-06-2021 |
| | | JP 2021514793 A | 17-06-2021 |
| | | JP 2021514794 A | 17-06-2021 |
| | | JP 2021514795 A | 17-06-2021 |
| | | US 2019274705 A1 | 12-09-2019 |
| | | US 2019274706 A1 | 12-09-2019 |
| | | US 2019274707 A1 | 12-09-2019 |
| | | US 2019274709 A1 | 12-09-2019 |
| | | US 2019274710 A1 | 12-09-2019 |
| | | US 2019274711 A1 | 12-09-2019 |
| | | US 2019274712 A1 | 12-09-2019 |
| | | US 2019274713 A1 | 12-09-2019 |
| | | US 2019274717 A1 | 12-09-2019 |
| | | US 2019274718 A1 | 12-09-2019 |
| | | US 2019274720 A1 | 12-09-2019 |
| | | US 2019274750 A1 | 12-09-2019 |
| | | US 2019274752 A1 | 12-09-2019 |
| | | WO 2019173137 A1 | 12-09-2019 |
| | | WO 2019173138 A1 | 12-09-2019 |
| | | WO 2019173141 A1 | 12-09-2019 |
| | | WO 2019173145 A1 | 12-09-2019 |

EPO FORM P0459

For more details about this annex : see Official Journal of the European Patent Office, No. 12/82

## ANNEX TO THE EUROPEAN SEARCH REPORT
## ON EUROPEAN PATENT APPLICATION NO.

EP 21 15 4898

This annex lists the patent family members relating to the patent documents cited in the above-mentioned European search report.
The members are as contained in the European Patent Office EDP file on
The European Patent Office is in no way liable for these particulars which are merely given for the purpose of information.

23-06-2021

| Patent document cited in search report | Publication date | Patent family member(s) | Publication date |
|---|---|---|---|
| | | WO 2019173151 A1 | 12-09-2019 |
| | | WO 2019173153 A1 | 12-09-2019 |
| | | WO 2019173191 A1 | 12-09-2019 |
| | | WO 2019173192 A1 | 12-09-2019 |
| | | WO 2019173193 A1 | 12-09-2019 |
| | | WO 2019173198 A1 | 12-09-2019 |
| | | WO 2019173200 A1 | 12-09-2019 |

EPO FORM P0459

For more details about this annex : see Official Journal of the European Patent Office, No. 12/82

page 3 of 3

EP 3 868 315 A1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 104582606 A
- CN 104582629 A
- CN 110507389 A
- CN 110448355 A
- CN 102458286 A
- CN 106999203 A
- CN 108366827 A
- CN 107205775 A
- CN 101772325 A